# FASCICULE DE BREVET EUROPEEN

(11) **EP 3 897 526 B1**
(45) Date de publication et mention de la délivrance du brevet: **22.02.2023**
(21) Numéro de dépôt: 19829121.3
(22) Date de dépôt: 18.12.2019
(51) Int. Cl.: A61K 8/19, A61K 8/26, A61K 8/365, A61K 8/368, A61K 8/49, A61K 8/9789, A61Q 1/10, A61K 8/81, A61K 8/04

(54) **GELS AQUEUX POUR LA COLORATION OU LE MAQUILLAGE DES SOURCILS**
WÄSSRIGE GELE ZUM FÄRBEN ODER SCHMINKEN DER AUGENBRAUEN
AQUEOUS GELS FOR DYEING OR MAKING UP THE EYEBROWS

(30) Priorité: 20.12.2018 FR 1873634
(43) Date de publication de la demande: 27.10.2021
(73) Titulaire: L V M H RECHERCHE, 45800 St. Jean de Braye (FR)
(72) Inventeur: LEGLAND, Lorraine, 45000 ORLEANS (FR); CHOISY, Patrick, 37370 Montlouis sur Loire (FR); PECHER, Virginie, 45380 La Chapelle Saint Mesmin (FR)
(74) Mandataire: Regimbeau
(86) Numéro de dépôt international: PCT/EP2019/085865
(87) Numéro de publication internationale: WO 2020/127434

(56) Documents cités:
- EP-A2- 2 196 188
- FR-A1- 2 994 082
- DATABASE GNPD [Online] MINTEL; 26 avril 2011 (2011-04-26), anonymous: "All Natural Pemanent Hair Color", XP055502858, extrait de www.gnpd.com Database accession no. 1522982

## Description

### DOMAINE DE L'INVENTION

La présente invention concerne le domaine de la coloration ou du maquillage des fibres kératiniques, en particulier des cils et/ou des sourcils et de préférence les sourcils.

### ETAT DE LA TECHNIQUE

On connaît de l'art antérieur des compositions de coloration ou de maquillage des matières kératiniques, en particulier la peau ou les cheveux comprenant des colorants naturels, en particulier des extraits végétaux comprenant des polyphénols. Les procédés de coloration utilisant des produits naturels, plus respectueux de la nature des fibres et de l'environnement, sont en effet de plus en plus appréciés des utilisateurs. Cependant le résultat coloration ou maquillage, en particulier lorsqu'il s'agit de fibres kératiniques, n'est pas toujours satisfaisant en terme d'intensité et/ou homogénéité et/ou tenue dans le temps.

Il subsiste donc le besoin de développer de nouvelles compositions et procédés de coloration ou maquillage des fibres kératiniques, en particulier des cils et/ou des sourcils et de préférence les sourcils, comprenant des colorants naturels à base de polyphénols, avec des propriétés chromatiques améliorées (intensité et homogénéité de la couleur) et qui soient résistantes aux lavages, à la transpiration et/ou au sébum.

La Demanderesse a justement découvert qu'il était possible d'améliorer l'efficacité de compositions de coloration ou maquillage des fibres kératiniques, en particulier des sourcils en mettant en oeuvre des extraits végétaux particuliers et des sels de fer et/ou sels d'aluminium, en particulier du gluconate de fer. Elle a notamment mis en évidence que l'utilisation d'agents boosters de pénétration spécifiques permettait d'améliorer l'intensité et la tenue de la couleur dans le temps.

La Demanderesse a également mis en évidence, pour des compositions de coloration ou maquillage sous forme de gels comprenant du gluconate de fer, que l'utilisation d'un copolymère acrylate particulier était avantageuse pour la stabilité et la texture des compositions.

### EXPOSE DE L'INVENTION

Un premier objet de l'invention est donc sur un procédé cosmétique de coloration ou maquillage des fibres kératiniques, en particulier les cils et/ou les sourcils et de préférence les sourcils, comprenant l'application sur lesdites fibres kératiniques, d'au moins :
a) Une composition cosmétique A colorante sous forme de gel aqueux comprenant, dans un milieu physiologiquement acceptable, un gélifiant polymérique acrylique, un ou plusieurs colorant(s) naturel(s) choisi(s) dans le groupe constitué par les néoflavonoïdes, les tanins galliques et tanins catéchiques, les proanthocyanidines et leurs dérivés, et leurs mélanges, ou de préférence un ou plusieurs extraits végétaux en contenant, et avantageusement un agent antioxydant,
b) Une composition cosmétique B révélatrice sous forme de gel aqueux comprenant, dans un milieu physiologiquement acceptable, un gélifiant polymérique choisi parmi les polymères anioniques associatifs, et du gluconate de fer,
la composition A étant appliquée avant ou après la composition B, de préférence avant la composition B.

L'invention porte également sur un kit ou dispositif de coloration ou maquillage des fibres kératiniques, en particulier les cils et/ou les sourcils et de préférence les sourcils, à plusieurs compartiments séparés et comprenant :
- dans un compartiment, la composition A colorante telle que définie selon l'invention ;
- dans un autre compartiment séparé la composition B révélatrice telle que définie selon l'invention et caractérisée en ce qu'elle ne comprend pas de sel de fer et/ou d'aluminium, en particulier de gluconate de fer, et
- dans un autre compartiment séparé, le sel de fer et/ou d'aluminium destiné à
   être mélangé extemporanément à la composition B révélatrice, et en particulier le gluconate de fer, avant
   application sur les fibres kératiniques,
- et avantageusement une notice d'utilisation.

Selon une alternative, le kit ou dispositif de coloration ou maquillage des fibres kératiniques, en particulier les cils et/ou les sourcils et de préférence les sourcils, à plusieurs compartiments séparés comprend :
- dans un compartiment, la composition A colorante telle que définie selon l'invention :
- dans un autre compartiment séparé la composition B révélatrice telle que définie selon l'invention et caractérisée en ce qu'elle comprend le sel de fer et/ou d'aluminium, en particulier du gluconate de fer,
- et avantageusement une notice d'utilisation.

Les kits selon l'invention pourront également comprendre, de façon optionnelle, dans un autre compartiment séparé une composition C comprenant un agent oxydant pour un pré-traitement des fibres kératiniques avant traitement par les compositions A et B. On sait en effet qu'un agent oxydant tel que le peroxyde d'hydrogène produit une légère altération des cuticules ou écailles, partie extérieure des fibres kératiniques, permettant une ouverture de celles-ci et par conséquent une meilleure pénétration des actifs. Dans le contexte de l'invention, l'utilisation d'un pré-traitement avec un agent oxydant permet de favoriser une meilleure pénétration des agents colorants.

### DESCRIPTION DETAILLEE DE L'INVENTION

La présente invention concerne donc un procédé cosmétique de coloration ou maquillage des fibres kératiniques, en particulier les cils et/ou les sourcils et de préférence les sourcils, comprenant l'application sur lesdites fibres kératiniques, d'au moins :
a) Une composition cosmétique A colorante sous forme de gel aqueux comprenant, dans un milieu physiologiquement acceptable, un gélifiant polymérique acrylique, un ou plusieurs colorant(s) naturel(s) choisi(s) dans le groupe constitué par les néoflavonoïdes, les tanins galliques et tanins catéchiques, les proanthocyanidines et leurs dérivés, et leurs mélanges, ou de préférence un ou plusieurs extraits végétaux en contenant, et avantageusement un agent antioxydant,
b) Une composition cosmétique B révélatrice sous forme de gel aqueux comprenant, dans un milieu physiologiquement acceptable, un gélifiant polymérique choisi parmi les polymères anioniques associatifs, et du gluconate de fer,
la composition A étant appliquée avant ou après la composition B, de préférence avant la composition B.

Par 'gel aqueux' selon l'invention, on entend que la composition est sous la forme d'une phase aqueuse continue gélifiée par au moins un gélifiant, de préférence un gélifiant polymérique. Les gélifiants polymériques de la composition A et respectivement de la composition B sont définis ci-après. La phase aqueuse contient généralement de l'eau, ou un mélange d'eau et d'un ou plusieurs solvants organiques. De préférence les compositions A et B utilisées dans le procédé selon l'invention comprennent chacune de l'eau ou un mélange d'eau et d'un ou plusieurs solvants organiques ou un mélange de solvants organiques.

A titre de solvant organique, on peut par exemple citer les mono-alcools en C₁-C₄, tels que l'éthanol et l'isopropanol ; les polyols et éthers de polyols comme le 2- butoxyéthanol, le propylèneglycol, le monométhyléther de propylèneglycol, le monoéthyléther et le monométhyléther du diéthylèneglycol, l'hexylène glycol, ainsi que les alcools aromatiques comme l'alcool benzylique ou le phénoxyéthanol. Les solvants organiques sont généralement présents dans les compositions de l'invention en des teneurs allant de 0,1 à 5%, de préférence de 1% à 2% en poids par rapport au poids total de la composition.

### Pré-traitement oxydant (optionnel)

Selon un premier mode de réalisation de l'invention, les compositions A et B ne contiennent pas d'agent oxydant et les fibres kératiniques ne sont pas traitées, avant ou après traitement par les compositions A et B, par un agent oxydant. Comme indiqué ci-dessus, ce pré-traitement oxydant favoriser l'ouverture des écailles des fibres kératiniques et permet ainsi de favoriser la pénétration des agents colorants.

Selon un autre mode de réalisation, on pré-traite les fibres kératiniques, en particulier les cils et/ou les sourcils et de préférence les sourcils, avec un agent oxydant, avant l'application desdites compositions A et B.

L'agent oxydant peut être choisi parmi le groupe formé par le peroxyde d'hydrogène, le peroxyde d'urée, les bromates ou ferricyanures de métaux alcalins, les sels peroxygénés comme par exemple les persulfates, les perborates, les peracides et leurs précurseurs et les percarbonates de métaux alcalins ou alcalino- terreux. De préférence on utilise le peroxyde d'hydrogène.

En particulier, on applique sur les fibres kératiniques, en particulier les cils et/ou les sourcils et de préférence les sourcils, une composition C de pré-traitement comprenant du peroxyde d'hydrogène comme agent oxydant, avant l'application des compositions colorante A et révélatrice B. La Demanderesse a en effet montré qu'un pré-traitement avec un agent oxydant permettait d'améliorer l'intensité et la tenue de la couleur dans le temps.

L'agent oxydant peut être présent dans la composition C de prétraitement en une teneur allant de 1 à 15% en poids, de préférence de 1 à 12% en poids par rapport au poids total de ladite composition.

En particulier, la composition comprend de 1 à 12% en poids de peroxyde d'hydrogène ou, en volumes, de 3 volumes à 40 volumes de la composition totale.

### Composition cosmétique A colorante

La composition cosmétique A colorante selon l'invention selon l'invention est sous forme d'un gel aqueux et comprend un ou plusieurs colorant(s) naturel(s) choisis dans le groupe constitué par les néoflavonoïdes, les tanins galliques et tanins catéchiques, les proanthocyanidines et leurs mélanges, et de préférence un ou plusieurs extraits végétaux en contenant.. En particulier, le ou les colorants naturels convenant à l'invention peuvent être choisis parmi l'hématoxyline, l'hématéine, la braziléine, la braziline, l'acide gallique, la catéchine, la castalagine, la vescalagine, les procyanidines, et leurs mélanges.

### Extraits végétaux comprenant des polyphénols

Les extraits végétaux utilisables selon l'invention sont des extraits végétaux comprenant au moins des polyphénols choisis dans le groupe constitué par les néoflavonoïdes, les tanins galliques et tanins catéchiques, les proanthocyanidines et leurs dérivés, et leurs mélanges. Ces polyphénols sont majoritaires dans la composition desdits extraits végétaux.

En particulier, les extraits végétaux convenant à l'invention sont choisis parmi les extraits végétaux contenant au moins un colorant choisi dans le groupe constitué par l'hématoxyline, l'hématéine, la braziléine, la braziline, l'acide gallique, la catéchine, la castalagine, la vescalagine, les procyanidines et leurs mélanges.

On peut notamment utiliser les extraits végétaux suivants (genre et espèces) :
- extraits de campêche (*Haematoxylon campechianum)* ou de brasiletto *(Haematoxylon brasiletto)* contenant notamment des tanins galliques (ou gallotanins) et des flavonoïdes/néoflavonoïdes ;
- extrait de châtaignier *(Castanea sativa)* contenant notamment des tanins hydrolysables de type ellagitanins comme la vescalagine et la castalagine et des tanins galliques tels que les monomères d'acide gallique et dérivés ;
- extraits de noix de Galle *(Rhus semialata gall extract)* ou d'écorce d'Anogeissus contenant notamment de l'elagitanine de type acide gallique et dérivés ;
- extraits de thé, de pin ou de vigne comprenant notamment des tanins condensés de type proanthocyanidines et en particulier catéchine et leurs dérivés catéchiques pour le thé et la vigne (OPC oligomeres procyanidoliques) ;
- extrait de cacao ;
- extrait de sorgho contenant notamment des anthocyanes et des tanins galliques et catéchiques ;
- extrait de myrobalan (Terminalia chebula fruit extract) contenant notamment de l'acide gallique
- extraits d'hibiscus et de fraisier contenant notamment des anthocyanes, et leurs mélanges.

Ainsi selon un mode particulier de l'invention, la composition A colorante comprend un colorant naturel choisi dans le groupe constitué par l'hématoxyline, l'hématéine, la braziléine, la braziline, l'acide gallique, la catéchine, la castalagine, la vescalagine, et/ou les procyanidines et leurs mélanges ou un extrait végétal en contenant, en particulier un extrait végétal choisi dans le groupe constitué par un extrait de campêche, un extrait de bois rouge, un extrait de châtaignier, un extrait de noix de Galle (*Rhus semialata gall extract*), un extrait d'écorce *d'Anogeissus* un extrait de pin, un extrait de thé, un extrait de vigne, un extrait de Sorgho, un extrait de cacao, un extrait de myrobalan, un extrait d'hibiscus, un extrait de fraisier et leurs mélanges, de préférence un extrait de campêche, un extrait de bois rouge, un extrait de châtaignier, et leurs mélanges.

Selon un mode particulier et préféré, la composition A colorante comprend au moins un extrait végétal choisi parmi un extrait de campêche, un extrait de châtaignier, un extrait de bois rouge, et leurs mélanges.

Les extraits sont obtenus par extraction de diverses parties de plantes, telles que par exemple la racine, le bois, l'écorce ou la feuille.

En particulier, on utilise un extrait issu des bois de campêche, de bois ou écorce de châtaignier, de feuille ou tige de sorgho, d'écorce de pin, de feuille de thé, de noix de Galle, de fève de cacao, de bois ou écorce de bois rouge et leurs mélanges.

Selon un premier mode particulier, la composition A colorante comprend au moins un extrait de campêche. En particulier, on utilise l'extrait de Campêche (*Hematoxylon Campechianum*) de la société SCRD ou 'Logwood extract' également connu sous la dénomination Hematin NO200. Il s'agit d'une poudre de couleur brun foncé à bordeaux comprenant 20-40% d'hématoxyline (polyphénol de type néoflavonoïde) en poids d'extrait sec. Il s'agit d'un colorant végétal pur d'extraction non oxydé obtenu à partir du bois de Campeche. Le degré d'oxydation provient de l'oxydation naturelle de l'Hématoxyline contenue dans le produit sans aucune oxydation chimique.

L'extrait de Campeche est constitué principalement du colorant Hématoxyline, des glucosides et tanins plus ou moins condensés. La structure chimique des deux principes colorants, Hématoxyline et Hématéine, permet leur classement dans le groupe des néoflavonoïdes. La gamme des couleurs que l'on peut obtenir avec L'extrait de Campeche est l'une des plus étendues de la teinture végétale.

Selon un autre mode particulier, la composition A colorante comprend au moins un extrait de bois ou écorce de châtaignier de nom INCI Castanea sativa (chestnut) bark extract.L'écorce et le bois de châtaignier contiennent notamment huit composés polyphénoliques, dont les ellagitanins, castaline, vescalagine, castalagine, acutissimine A, kurigaline, chestanine. On peut notamment utiliser un extrait de bois de châtaignier obtenu par extraction aqueuse du bois, filtration, concentration et séchage et tel que commercialisé par la société Couleurs de plantes.

Selon un autre mode particulier, la composition A colorante comprend au moins un extrait de bois ou écorce de Brasiletto (ou bois rouge). Le Bois Rouge (*Haematoxylum Brasiletto ou Brasilensis*) est un petit arbre (2 à 12 mètres) communément présent dans toute l'Amérique Centrale. L'extrait de bois de Brasiletto ou bois rouge est riche en Bréziline (sa forme oxydéee est la Braziléine). On peut utiliser notamment un extrait de Brasiletto commercialisé par la société SCRD.

La composition A colorante peut également comprendre un mélange d'extraits végétaux selon l'invention, en fonction de la teinte recherchée. Selon un mode particulier, la composition A colorante comprend un mélange d'extrait de campêche et d'extrait de châtaignier.

Le ou les colorants naturels ou le ou les extraits végétaux en contenant sont présents dans la composition A colorante de l'invention en une teneur totale allant de 0,1 à 10%, de préférence de 2 à 5% en poids par rapport au poids total de la composition. L'homme du métier saura adapter la teneur totale en extraits végétaux à utiliser dans la composition en fonction de l'effet recherché et de la nature et des teneurs en colorant(s) naturel(s) desdits extraits.

### Agent anti-oxydant (agent réducteur d'oxydation)

La composition A colorante comprend également avantageusement au moins un agent antioxydant (autrement nommé agent réducteur d'oxydation). Cet agent antioxydant permet à la fois de protéger l'extrait végétal de l'oxydation et d'agir au niveau de la fibre kératinique et en particulier du sourcil pour casser les ponts disulfures et améliorer ainsi la coloration.

L'agent antioxydant peut être choisi notamment parmi les sulfites, les bisulfites, les thiols et les phosphines, de préférence les sulfites et bisulfites de métaux alcalins ou alcalino-terreux. On peut citer en particulier le sulfite ou le bisulfite de sodium.

Selon un mode particulier et préféré, la composition A colorante comprend un bisulfite de sodium (sodium métabisulfite) comme agent antioxydant.

L'agent antioxydant pourra être présent dans la composition A colorante en une teneur allant de 0,01 à 0,5%, de préférence de 0,05 à 0,1% en poids par rapport au poids total de ladite composition

### Agent favorisant et/ou augmentant la pénétration du colorant (autrement nommé `booster de pénétration')

Selon un mode particulier et préféré de l'invention, la composition A comprend en outre un agent favorisant et/ou augmentant la pénétration du colorant naturel dans les fibres kératiniques, en particulier les cils et/ou les sourcils et de préférence les sourcils.

La Demanderesse a en effet montré, comme illustré dans les exemples ci-après que l'utilisation de certains agents augmentant la pénétration du colorant naturel dans la fibre kératinique, permettait d'améliorer les propriétés de coloration ou sa tenue dans le temps. On parle indifféremment d'agent favorisant et/ou augmentant la pénétration du colorant naturel ou agent 'booster' de pénétration du colorant naturel.

Cet agent favorisant et/ou augmentant la pénétration du colorant naturel dans la fibre kératinique et en particulier le sourcil, est distinct de l'alcool benzylique.

Cet agent favorisant et/ou augmentant la pénétration du colorant naturel dans la fibre kératinique et en particulier le sourcil est notamment choisi parmi les éthers bicycliques, les esters d'acides gras comprenant des chaînes hydrocarbonées en C6 à C18 polyéthoxylées, et leurs mélanges.

Selon un mode particulier et préféré, l'agent favorisant et/ou augmentant la pénétration du colorant naturel dans la fibre kératinique est choisi parmi des éthers bicycliques, chaque cycle du bicycle comprenant un nombre n de chaînons compris entre 3 et 10, les esters d'acides gras comportant une chaîne hydrocarbonée en C6 à C18, saturée ou insaturée, linéaire ou ramifiée à l'aide d'autres chaînes hydrocarbonées en C6 à C18, chacune des chaînes hydrocarbonées étant polyéthoxylée, avec un degré d'éthoxylation d'au moins 2, notamment de 2 à 12, et leurs mélanges. Selon un mode particulier et préféré, l'éther bicyclique est le dimethyl isosorbide. Selon un autre mode particulier et préféré, l'ester d'acide gras polyethoxylé est le PEG-7 glyceryl cocoate.

Selon un mode particulier, la composition A colorante comprend au moins du diméthyl isosorbide, tel que celui commercialisé sous les dénominations ARLASOLVE^{™} DMI ou GRANSOLVE^{™} DMI par la société CRODA, Dottisol^{™} par la société Dottikon ES AG, NEX-DMI^{™} par la société Nexgen Biotechnologies, Inc ; ou OriStar DMI^{™} par la société Orient Stars LLC.

Selon un autre mode particulier, la composition A colorante comprend au moins du PEG-7 glyceryl cocoate, tel que celui commercialisé sous les dénominations CETIOL^{®} HE par la société BASF, ou Chemonic LI-7 Surfactant par la société Lubrizol Advanced Materials, Inc, ou Tegosoft GC par la société Evonik Nutrition & Care GmbH ou Nikkol SG-CG700 par la société Nikko Chemicals Co., Ltd ou Glycerox HE par la société Croda.

La composition A colorante peut également comprendre des alcools aromatiques notamment choisis parmi l'alcool benzylique, le phényl éthanol et le phényl propanol, comme agents conservateurs. Selon un mode particulier, la composition A colorante comprend en outre du phénoxyéthanol et de l'alcool benzylique à titre d'agents conservateurs.

La teneur en agents conservateurs pourra aller de 0,1 à 5%, en particulier de 0,3 à 3% et de préférence de 0,5 à 2% en poids par rapport au poids total de ladite composition.

### Tensioactif non ionique

La composition A colorante comprend en outre avantageusement un tensioactif non ionique, en particulier un alkyl glucoside, de préférence le decyl glucoside.

Comme tensioactif non ionique utilisable dans la composition A colorante de l'invention, on peut citer notamment les alkylpolyglucosides.

Selon un mode particulier, on utilisera un alkyl glucoside comme tensioactif non ionique selon l'invention. Ces tensio-actifs connus de l'état de la technique peuvent être plus représentés par la formule générale suivante : R₁O-(R₂O)ₜ (G)ᵥ dans laquelle
- R1 représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone,
- R2 représente un radical alkylène comportant environ de 2 à 4 atomes de carbone,
- G représente un motif sucre comportant de 5 à 6 atomes de carbone,
- t désigne une valeur allant de 0 à 10, de préférence 0 à 4, de préférence 0 à 4 et
- v désigne une valeur allant de 1 à 15.

Selon un mode de réalisation particulier, les tensio-actifs alkylpolyglucosides sont des composés de formule décrite ci-dessus dans laquelle R1 désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule ci-dessus, peut aller de 1 à 15, de préférence de 1 à 4. Le degré moyen de polymérisation est plus particulièrement compris entre 1 et 2. Les liaisons glucosidiques entre les motifs sucre sont de type 1-6 ou 1-4 et de préférence 1-4.

Des composés correspondant à la formule ci-dessus sont notamment représentés par les produits vendus par la société BASF sous les dénominations PLANTAREN^{®} (600 CS/U, 1200 et 2000) ou PLANTACARE^{®} (818, 1200 et 2000) ou LUTENSOL GD 70. On peut également utiliser les produits vendus par la société SEPPIC sous les dénominations TRITON CG110 (ou ORAMIX CG 10) et TRITON CG312 (ou ORAMIX^{®} NS 10).

Selon un mode particulier et préféré, la composition A colorante comprend un decyl glucoside tel que celui commercialisé sous la dénomination PLANTACARE 2000^{®} UP ou PLANTACARE 2000^{®} UP/MB par la société BASF, ou Triton CG-50 Surfactant par la société Dow Chemical Company, ou Blanova Tens APG 2000 par la société Azelis Deutschland Kosmetik GmbH ou Oramix NS 10 par la société Seppic.

La teneur en tensioactif non ionique, de préférence en alkylpolyglucoside et de préférence encore en decyl glucoside dans la composition A colorante de l'invention ira de 0,5 à 10% en poids, de préférence de 3% à 5% en poids par rapport au poids total de la composition A.

### Gélifiant polymérique acrylique

La composition A colorante comprend au moins un gélifiant polymérique acrylique, en particulier choisi parmi les polymères ou copolymères d'acides acryliques et méthacryliques comme les copolymères acide acrylique/acrylate d'éthyle et les polymères carboxyvinyliques.

La présence de ce gélifiant polymérique acrylique permet de structurer la composition en augmentant sa viscosité.

Le gélifiant polymérique selon l'invention est un gélifiant polymérique acrylique choisi parmi les polymères carboxyvinyliques modifiés ou non. Ces polymères carboxyvinyliques peuvent être des copolymères issus de la polymérisation d'au moins un monomère (a) choisi parmi les acides carboxyliques à insaturation α,β-éthylénique ou leurs esters, avec au moins un monomère (b) à insaturation éthylénique comportant un groupement hydrophobe. On entend par « *copolymères »* aussi bien les copolymères obtenus à partir de deux sortes de monomères que ceux obtenus à partir de plus de deux sortes de monomères tels que les terpolymères obtenus à partir de trois sortes de monomères.

Leur structure chimique comprend plus particulièrement au moins un motif hydrophile et au moins un motif hydrophobe. Par groupement ou motif hydrophobe, on entend un radical à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

Ces gélifiants polymériques sont par exemple les polymères ou copolymères d'acides acryliques et méthacryliques comme les copolymères acide acrylique/acrylate d'éthyle et les polymères carboxyvinyliques. Des exemples de tels polymères ou copolymères sont notamment les "carbomer" (CTFA) vendus sous la dénomination Carbopol^{®} par la société GOODRICH ou par la société LUBRIZOL. On peut encore citer le polyglycérylméthacrylate commercialisé par la société GUARDIAN sous la dénomination Lubragel ou encore le polyglycérylacrylate commercialisé sous la dénomination Hispagel par la société HISPANO CHIMICA ou enfin le mélange polyacrylamide/C1 3-C14 Isoparaffin/Laureth7 commercialisé par la société SEPPIC sous la dénomination Sepigel.

Selon un mode particulier, la composition A colorante comprend un homopolymère d'acide polyacrylique de nom INCI Carbomer.

La teneur en gélifiant polymérique acrylique dans la composition A colorante de l'invention ira généralement de 0,1% à 5% en poids, de préférence de 1% à 2% en poids par rapport au poids total de la composition

### Matières colorantes additionnelles

La composition A colorante de l'invention pourra comprendre en outre avantageusement au moins une matière colorante additionnelle choisie parmi les pigments, les colorants et leurs mélanges.

L'utilisation de ces pigments et/ou colorants additionnels permet notamment d'obtenir davantage de nuances de couleurs.

La ou les matière(s) colorante(s) peuvent être choisies parmi les matières colorantes hydrosolubles ou non, liposolubles ou non, organiques ou inorganiques, les matériaux à effet optique, et leurs mélanges. On entend par matière colorante au sens de la présente invention, un composé susceptible de produire un effet optique coloré lorsqu'il est formulé en quantité suffisante dans un milieu cosmétique approprié.

Selon un mode particulier, la ou les matières colorantes sont notamment choisies parmi des pigments minéraux et/ou organiques, des pigments composites (à base de matériaux minéraux et/ou organiques), des colorants, et leurs mélanges.

Par pigments, il faut comprendre des particules colorées, inorganiques (minérales) ou organiques, insolubles dans la phase organique liquide. Parmi les pigments minéraux, on peut citer, à titre d'exemples les oxydes de fer noir, jaune, rouge et brun ; le violet de manganèse ; le bleu outremer, l'oxyde de chrome, l'oxyde de chrome hydraté et le bleu ferrique.

Parmi les pigments organiques, on peut citer, par exemple, les pigments D & C red n° 19; D & C red n° 9; D & C Red n° 22 ; D & C Red n° 21 ; D & C Red n° 28 ; D & C Yellow n° 6 ; D & C orange n° 4 ; D & C orange n° 5 ; D & C Red n° 27; D & C red n° 13; D & C Red n° 7 ; D & C Red n° 6 ; D & C Yellow n° 5; D & C Red n° 36 ;; D & C Red n° 33 ; D & C orange n° 10; D & C yellow n° 6 ; ; D & C Red n° 30 ; D &C red n° 3 ; D &C Blue 1 ; le noir de carbone et les laques à base de carmin de cochenille.

Parmi les colorants hydrosolubles, on pourra citer Yellow 5, Yellow 6, Blue 1, Green 5, Green 3, Green 6, Orange 4, Red 4, Red 21,Red 22, Red 27, Red 28, Red 33, Red 40, le carminé de cochenille (CI 15850, CI 75470).

Selon un mode particulier, la composition A colorante comprend au moins une matière colorante choisie parmi les colorants directs, de préférence les colorants cationiques directs.

Ces colorants directs sont par exemple choisis parmi tous les colorants aromatiques et/ou non aromatiques d'utilisation courante tels que les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents.

Selon un mode particulier et préféré, la composition A colorante comprendra un ou plusieurs colorants directs cationiques. On peut citer notamment les colorants directs cationiques de nom INCI suivants : BASIC RED 76, BASIC YELLOW 57, HC BLUE NO. 15, et leurs mélanges.

La teneur en matières colorantes additionnelles, notamment en colorants directs, en particulier en colorants cationiques directs, dans la composition A colorante de l'invention, ira généralement de 0,005 à 5% en poids, de préférence de 0,005% à 0,5% en poids par rapport au poids total de la composition

### Composition cosmétique B révélatrice

La composition B révélatrice de la couleur selon l'invention est sous forme d'un gel aqueux et comprend au moins un sel de fer et/ou sel d'aluminium. Les sels de fer en particulier permettent d'obtenir des teintes foncées recherchées allant du marron au noir. Ces sels permettent la complexation métallique sur la fibre kératinique, et en particulier le sourcil.

Selon l'invention, on utilisera du gluconate de fer.

### Sels de fer et/ou sels d'aluminium selon la description et selon l'invention

Par « sels de fer et/ou sels d'aluminium», on entend selon la description les oxydes de ces métaux et les sels proprement dits issus notamment de l'action d'un acide sur un métal. De préférence les sels ne sont pas des oxydes. Parmi les sels on peut citer les halogénures tels que les chlorures, fluorures et iodures ; les sulfates, les phosphates ; les nitrates ; les perchlorates et les sels d'acides carboxyliques et les complexes polymériques pouvant supporter lesdits sels, ainsi que leurs mélanges. Les sels d'acides carboxyliques utilisables selon la description incluent également des sels d'acides carboxyliques hydroxyles tels que le gluconate.

Parmi les sels de fer ou d'aluminium selon la description, on peut citer les sulfates, les gluconates, les chlorures, les lactates, les acétates, les glycinates, les aspartates, et les citrates.

On peut également citer, comme sels d'aluminium, un ou plusieurs alun(s), c'est-à-dire un ou plusieurs sulfates mixtes d'aluminium et de cation monovalent, choisis notamment parmi le sulfate d'aluminium et de potassium (alun de potassium), le sulfate d'aluminium et de sodium (alun de sodium), et le sulfate d'aluminium et d'ammonium (alun d'ammonium).

De préférence, on peut citer le gluconate de fer, les aluns de potassium, de sodium ou d'ammonium, et les mélanges de ces sels, de préférence encore le gluconate de fer.

Selon l'invention, la composition B révélatrice comprend au moins un gluconate de fer. On peut citer notamment le gluconate de fer commercialisé par la société Givaudan-Lavirotte sous la dénomination GIVOBIO Fe 601 ou Gluconal FE de la société Glucona America, Inc.

Le ou les sels de fer et/ou sels d'aluminium selon la description, et de préférence le gluconate de fer selon l'invention, utilisés dans la composition B révélatrice représentent avantageusement de 0,1% à 10%, de préférence de 3% à 5% en poids du poids total de la composition.

Le sel de fer et/ou le sel d'aluminium, de préférence le gluconate de fer ou un sulfate de fer, de préférence encore le gluconate de fer selon l'invention, peut être intégré dans la composition B révélatrice, mais de préférence il est ajouté extemporanément à la composition B révélatrice, avant application de celle-ci sur lesdites fibres kératiniques, en particulier les cils et/ou les sourcils et de préférence les sourcils

### Gélifiant polymérique- Polymères associatifs

La composition B révélatrice sous forme d'un gel aqueux comprend un gélifiant polymérique hydrophile choisi parmi les polymères associatifs.

Ce gélifiant polymérique dans une composition B révélatrice sous forme de gel permet d'améliorer la stabilité de la composition, en particulier au contact du gluconate de fer, susceptible d'impacter la stabilité et/ou la structure de ladite composition.

Des exemples de polymères associatifs, en particulier de polymères associatifs anioniques convenant à l'invention sont décrits ci-après dans le chapitre galénique.

Le polymère gélifiant hydrophile, en particulier le polymère associatif anionique présent dans la composition B révélatrice, sera présent en une teneur allant de 0,1% à 5% en poids, de préférence de 0,5% à 3% voire de 1% à 3% en poids par rapport au poids total de ladite composition.

### Polymère filmogène

Selon un mode particulier, la composition B révélatrice comprend au moins un polymère filmogène.

Ce polymère filmogène permet d'améliorer la tenue de la coloration ou du maquillage sur les matières kératiniques, en particulier les cils et/ou les sourcils et de préférence les sourcils.

Ce polymère filmogène peut notamment être choisi parmi les polymères cationiques, anioniques, amphotères, ou non ioniques utilisés classiquement dans le domaine capillaire, ou parmi les polymères filmogènes utilisés dans le domaine des mascaras.

Selon un mode particulier, le polymère filmogène est choisi parmi les copolymères acryliques.

En particulier, on peut citer une dispersion aqueuse de polymère filmogène non-ionique (méth)acrylate d'alkyle en C1-C6, et notamment de polymère consistant essentiellement en un ou plusieurs (méth)acrylates d'alkyle en C1-C6. Ces polymères peuvent être choisis parmi les polymères de (méth)acrylate d'alkyle en C1-C4, et en particulier les copolymères d'acrylate d'alkyle en C1-C4 et de méthacrylate d'alkyle en C1-C4, et leurs mélanges. Comme (méth)acrylate d'alkyle en C1-C4, on peut citer le (méth)acrylate de méthyle, le (méth)acrylate d'éthyle, le (méth)acrylate de propyle, le (méth)acrylate de butyle. De préférence, on peut utiliser un copolymère d'acrylate d'éthyle et de méthacrylate de méthyle tel que celui vendu sous la dénomination "DAITOSOL 5000 AD" par la société DAITO KASEY KOGYO.

Comme autres exemples de polymères utilisables selon l'invention :
- Le copolymère ACRYLATES/ETHYLHEXYL ACRYLATE vendu sous la dénomination de Daitosol 5000 SJT ^{®} par la société Daito Kasei,
- Le copolymère SODIUM STYRENE/ACRYLATES COPOLYMER vendu sous la dénomination de Daitosol 5000 STY ^{®} par la société Daito Kasei,
- Le copolymère ACRYLATES/ETHYLHEXYL ACRYLATE COPOLYMER vendu sous la dénomination de Daitosol 4000 SJT par la société Daito Kasei,
- Le copolymère ACRYLATES vendu sous la dénomination de Daitosol 5000 AD^{®} par la société Daito Kasei.

Le polymère filmogène utilisé avantageusement dans la composition B révélatrice peut être présent en une teneur allant de 1% à 10%, de préférence de 1% à 5% en poids (d'extrait sec de polymère) du poids total de la composition.

### GALENIQUE

Les différentes compositions de l'invention décrites ci-dessus sont chacune sous la forme d'un gel aqueux.

Elles seront préférentiellement appliquées sur les fibres kératiniques, en particulier les cils et/ou les sourcils, et de préférence les sourcils, à l'aide d'un pinceau ou d'une brosse.

Les compositions utilisées dans le procédé selon l'invention comprennent une phase aqueuse pouvant contenir de l'eau, un mélange d'eau et d'un ou plusieurs solvants organiques.

De préférence les compositions A et B utilisées dans le procédé selon l'invention comprennent chacune de l'eau ou un mélange d'eau et d'un ou plusieurs solvants organiques ou un mélange de solvants organiques.

A titre de solvant organique, on peut par exemple citer les mono-alcools en C₁-C₄, tels que l'éthanol et l'isopropanol ; les polyols et éthers de polyols comme le 2- butoxyéthanol, le propylèneglycol, le monométhyléther de propylèneglycol, le monoéthyléther et le monométhyléther du diéthylèneglycol, l'hexylène glycol, ainsi que les alcools aromatiques comme l'alcool benzylique ou le phénoxyéthanol. Les solvants organiques sont généralement présents dans les compositions de l'invention en des teneurs allant de 0,1 à 5%, de préférence de 1% à 2% en poids par rapport au poids total de la composition.

Les compositions de l'invention peuvent également renfermer divers adjuvants tels que des agents antioxydants, des agents séquestrants, des parfums, des ajusteurs de pH autrement nommés acidifiants ou alcalinisants, des agents dispersants, des agents de conditionnement et leurs mélanges.

### Ajusteurs de pH (pour compositions A et/ou B)

La composition A colorante et/ou la composition B révélatrice comprennent avantageusement un ajusteur de pH.

De préférence, le pH desdites compositions iront de 6 à 8,5, de préférence de 7 à 8. Selon un mode particulier, le pH des compositions va de 7 à 7,5.

Le pH des compositions utilisées dans le procédé selon l'invention peut ainsi être ajusté à la valeur désirée au moyen d'agents acidifiants ou alcalinisants habituellement utilisés en cosmétique ou bien encore à l'aide de systèmes tampons classiques. Parmi les agents acidifiants des compositions utilisées dans l'invention, on peut citer, à titre d'exemple, l'acide citrique.. Une variante avantageuse est d'ajouter un agent alcalinisant à la ou les compositions du procédé selon l'invention, contenant le ou les (bi)carbonates. Plus particulièrement, cet agent alcalin est choisi parmi l'ammoniaque, les carbonates ou bicarbonates alcalins tels que les carbonates ou bicarbonates de sodium ou de potassium, les alcanolamines telles que les mono-, di- et triéthanolamines ainsi que leurs dérivés, les hydroxydes de sodium ou de potassium, et leurs mélanges. L'homme du métier saura choisir l'ajusteur de pH adapté à la zone d'application du sourcil (contour de l'oeil).

Selon un mode particulier et préféré de l'invention, la ou les compositions de l'invention comprennent des bicarbonates alcalins, en particulier du bicarbonate de sodium.

Les gels aqueux selon l'invention sont caractérisés par une phase aqueuse comprenant généralement de l'eau et des solvants organiques hydrosolubles tels que décrits précédemment, et un ou plusieurs gélifiants, de préférence des gélifiants polymériques acryliques.

La présence de ces gélifiants polymériques permet de structurer les compositions A et B en augmentant leur viscosité. De préférence, la viscosité des compositions A et B ira de 2000cps à 10 000cps, de préférence de 4000cps à 7000cps, mesurée au Rhéolab avec un mobile Ailette.

La composition A colorante comprend un gélifiant polymérique acrylique, tel que décrit précédemment.

Ces gélifiants polymériques acryliques sont par exemple les polymères ou copolymères d'acides acryliques et méthacryliques comme les copolymères acide acrylique/acrylate d'éthyle et les polymères carboxyvinyliques. Des exemples de tels polymères ou copolymères sont notamment les "carbomer" (CTFA) vendus sous la dénomination Carbopol^{®} par la société GOODRICH ou par la société LUBRIZOL. On peut encore citer le polyglycérylméthacrylate commercialisé par la société GUARDIAN sous la dénomination Lubragel ou encore le polyglycérylacrylate commercialisé sous la dénomination Hispagel par la société HISPANO CHIMICA ou enfin le mélange polyacrylamide/C1 3-C14 Isoparaffin/Laureth7 commercialisé par la société SEPPIC sous la dénomination Sepigel.

Selon un mode particulier, la composition A colorante comprend un homopolymère d'acide polyacrylique de nom INCI Carbomer.

La teneur en gélifiant polymérique dans la composition A colorante de l'invention ira généralement de 0,1% à 3%% en poids, de préférence de 1% à 2%% en poids par rapport au poids total de la composition

La composition B révélatrice comprend un gélifiant polymérique hydrophile choisi parmi les polymères associatifs.

Ce gélifiant polymérique dans une composition B révélatrice sous forme de gel permet d'améliorer la stabilité de la composition, en particulier au contact du gluconate de fer, susceptible d'impacter la stabilité et/ou la structure de ladite composition.

Selon un mode particulier et préféré, la composition B révélatrice sous forme de gel aqueux comprend au moins un polymère associatif.

Par *« polymère associatif »* selon la présente invention, on entend tout polymère amphiphile comportant dans sa structure au moins une chaîne grasse et au moins une portion hydrophile. Les polymères associatifs conformes à la présente invention peuvent être anioniques, cationiques, non-ioniques ou amphotères. De préférence, il s'agira d'un polymère anionique associatif.

Parmi les polymères anioniques associatifs, on peut citer ceux comportant au moins un motif hydrophile, et au moins un motif éther d'allyle à chaîne grasse, plus particulièrement parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyle à chaîne grasse correspond au monomère de formule (I) suivante :

CH₂ = C(R')CH₂ OBₙR (I)

dans laquelle :
- R' désigne H ou CH₃,
- B désigne le radical éthylèneoxy,
- n est nul ou désigne un entier allant de 1 à 100,
- R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone.

Parmi les polymères anioniques associatifs, on peut citer également les terpolymères d'anhydride maléique/cc-oléfine en C₃₀-C₃₈/maléate d'alkyle tel que le produit copolymère anhydride maléique/cc-oléfine en C₃₀-C₃₈/maléate d'isopropyle vendu sous le nom PERFORMA V 1608 par la société NEWPHASE TECHNOLOGIES.

Parmi les polymères anioniques associatifs on peut citer selon un mode de réalisation préféré, les copolymères comportant parmi leurs monomères un acide carboxylique à insaturation a,(3-monoéthylénique et un ester d'acide carboxylique à insaturation α,β-monoéthylénique et d'un alcool gras oxyalkyléné. Préférentiellement, ces composés comprennent également comme monomère un ester d'acide carboxylique à insaturation α,β-monoéthylénique et d'alcool en C1-C4.

A titre d'exemple de ce type de composé on peut citer l'ACULYN 22^{®} vendu par la société ROHM et HAAS, qui est un terpolymère acide méthacrylique/acrylate d'éthyle/méthacrylate de stéaryle oxyalkyléné (comprenant 20 motifs OE) ou l'ACULYN 28 (terpolymère d'acide méthacrylique/acrylate d'éthyle/méthacrylate de béhényle oxyéthylène (25 OE).

Comme polymères anioniques associatifs, on peut citer également les polymères anioniques comportant au moins un motif hydrophile de type acide carboxylique insaturé oléfinique, et au moins un motif hydrophobe exclusivement de type ester d'alkyl (C10-C30) d'acide carboxylique insaturé.

Comme polymères anioniques associatifs, on peut citer également les terpolymères anioniques.

Le terpolymère anionique peut être un terpolymère linéaire ou branché et/ou réticulé, d'au moins un monomère (1) portant une fonction acide sous forme libre, partiellement ou totalement salifiée avec un monomère non-ionique (2) choisi parmi le NN,dimethylacrylamide et l'acrylate de 2-hydroxyéthyle et au moins un monomère (3) acrylate d'alkyle polyoxyéthyléné de formule (II) suivante : 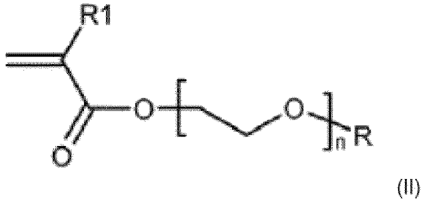 dans laquelle R1 représente un atome d'hydrogène, R représente un radical alkyle linéaire ou ramifié en C₂-C₈ et n représente un nombre allant de 1 à 10.

Par *«polymère branché »,* on désigne un polymère non linéaire qui possède des chaînes pendantes de manière à obtenir, lorsque ce polymère est mis en solution dans l'eau, un fort état d'enchevêtrement conduisant à des viscosités, à bas gradient de vitesse très importantes.

Par *«polymère réticulé »,* on désigne un polymère non linéaire se présentant à l'état de réseau tridimensionnelle insoluble dans l'eau mais gonflable à l'eau et conduisant à l'obtention d'un gel chimique.

La fonction acide du monomère (1) est notamment la fonction acide sulfonique, acide phosphonique, lesdites fonctions étant sous forme libre, partiellement ou totalement salifiées.

Le monomère (1) peut être choisi parmi l'acide styrènesulfonique, l'acide éthylsulfonique ou l'acide 2-méthy1-2[(1-oxo-2-propènyl]amino] 1-propanesulfonique (appelé aussi Acryloyldimethyltaurate) sous forme libre, partiellement ou totalement salifiée. Il est présent dans le terpolymère anionique de préférence dans des proportions molaires comprises entre 5% et 95% molaire et plus particulièrement entre 10% et 90% molaire. Le monomère (1) sera plus particulièrement l'acide 2-méthyl-2-[(1-oxo-2- propènyl]amino] 1-propanesulfonique sous forme libre, partiellement ou totalement salifiée.

La fonction acide sous forme partiellement ou totalement salifiée sera de préférence un sel de métal alcalin tel qu'un sel de sodium ou de potassium, un sel d'ammonium, un sel d'aminoalcool comme un sel de monoéthanolamine ou bien un sel d'acide aminé tel qu'un sel de la lysine.

Le monomère (2) est de préférence présent dans le terpolymère anionique dans des proportions molaires comprises entre 4,9% et 90% molaire et plus particulièrement entre 9,5% et 85% molaire et encore plus particulièrement entre 19,5% et 75% molaire.

Dans la formule (II), comme exemple de radical alkyle linéaire en C₈-C₁₆, on peut citer octyle, décyle, undécyle, tridécyle, tétradécyle, pentadécyle, hexadécyle.

Dans la formule (II), comme exemple de radical alkyle ramifié en C₈-C₁₆, on peut citer 2-éthylhexyle, 2-propylheptyle, 2-butyloctyle, 2-pentylnonyle, 2hexyldécyle, 4- méthylpentyle, 5-méthylhexyle, 6-méthylheptyle,15-méthylpentadécyle, 16- méthylheptadécyle, 2-hexyloctyle.

Selon une forme particulière de l'invention, dans la formule (I), R désigne un radical alkyle en C12-C16.

Selon une forme particulière de l'invention, dans la formule (I), n varie de 3 à 5.

On utilisera plus particulièrement comme monomère de formule (II) l'acrylate de lauryle tétraéthoxylé.

Le monomère (3) de formule (II) est de préférence présent dans le terpolymère anionique dans des proportions molaires comprises entre 0,1% et 10% molaire et plus particulièrement entre 0,5% et 5% molaire.

Selon un mode particulier de l'invention, le terpolymère anionique est réticulé et/ou branché par un composé diéthylénique ou polyéthylénique dans la proportion exprimée par rapport à la quantité totale de monomères mis en oeuvre, de 0,005% à 1% molaire et de préférence de 0,01% à 0,5% molaire et plus particulièrement de 0,01% à 0,25% molaire.

L'agent de réticulation et/ou l'agent de ramification est choisi de préférence parmi le diméthacrylate d'éthylèneglycol, l'acide diallyloxoacétique ou un de ses sels, comme le diallyloxyacétate de sodium, le tétraallyloxyéthane, le diacrylate d'éthylèneglycol, le diallyl uré, la triallyl amine, le triméthylol propanetriacrylate, le méthylènebis-(acrylamide) ou leurs mélanges.

Le terpolymère anionique peut contenir des additifs tels que des agents complexants, des agents de transfert, des agents limiteurs de chaîne.

On utilisera plus particulièrement dans la composition B révélatrice de l'invention un terpolymère anionique d'acide 2-méthyl-2-[(1-oxo-2-propènyl]amino] 1-propanesulfonique partielllement ou totalement salifié sous forme de sel d'ammonium, de N,N-diméthylacrylamide et d'acrylate de lauryle tétraéthoxylé et réticulé au triméthylol propanetriacrylate, de nom INCI Polyacrylate Crosspolymer-6 tel que le produit vendu sous le nom commercial SEPIMAX ZEN^{®} par la société SEPPIC.

Le polymère gélifiant hydrophile, en particulier le polymère associatif anionique présent dans la composition B révélatrice, sera présent en une teneur allant de 0,1% à 5% en poids, de préférence de 0,5% à 3% voire de 1% à 3% en poids par rapport au poids total de ladite composition.

### Mise en oeuvre du procédé de coloration ou maquillage

Les compositions A et B peuvent être appliquées sur les fibres kératiniques, en particulier les cils et/ou les sourcils, de préférence les sourcils, séparément et en particulier de façon séquentielle, quel que soit leur ordre d'application (application de la composition A puis de la composition B ou application de la composition B puis de la composition A). Selon un mode particulier et préféré, la composition A colorante est appliquée avant la composition B révélatrice.

Les fibres kératiniques, de préférence les sourcils, peuvent être ou non préalablement humidifiées. De préférence, on appliquera la composition A colorante sur des fibres kératiniques non préalablement humidifiées.

Les fibres kératiniques, de préférence les sourcils, peuvent être ou non préalablement traitées par un agent oxydant. Selon un premier mode, on appliquera la composition A colorante sur des fibres kératiniques qui ne sont pas préalablement traitées par un agent oxydant. Selon un autre mode, on appliquera la composition A colorante sur des fibres kératiniques préalablement traitées par un agent oxydant, en particulier du peroxyde d'hydrogène.

L'application des compositions A et B peut être précédée ou suivie d'une étape de rinçage et/ou de séchage des fibres kératiniques. Selon un mode particulier, le procédé ne comprend pas d'étape de rinçage et/ou de séchage avant l'application des compositions A et B. Selon un mode préféré, le procédé comprend néanmoins une étape de rinçage final après application de la composition B.

Le temps de pose après application de la composition A colorante varie généralement de 30 secondes à 30 minutes, préférentiellement de 45 secondes à 10 minutes, et plus préférentiellement de 1 minute à 6 minutes.

Le temps de pose après application de la composition B révélatrice varie généralement de 30 secondes à 10 minutes, préférentiellement de 45 secondes à 5 minutes.

Les compositions A et B sont généralement appliquées à température ambiante.

Ainsi, l'invention porte également sur un procédé cosmétique de coloration ou de maquillage des fibres kératiniques, en particulier des cils et/ou des sourcils et de préférence des sourcils, dans lequel :
- la composition A colorante est appliquée sur les fibres kératiniques, en particulier les cils et/ou les sourcils et de préférence les sourcils, avec un temps de pose allant de 30 secondes à 30 minutes, préférentiellement de 45 secondes à 10 minutes, préférentiellement de 1 minutes à 6 minutes,
- la composition B révélatrice est appliquée les fibres kératiniques, en particulier les cils et/ou les sourcils et de préférence les sourcils, avec un temps de pose allant de 30 secondes à 10 minutes, préférentiellement de 45 secondes à 5 minutes,
   la composition A étant appliquée avant ou après la composition B, de préférence avant la composition B,
- lesdites fibres kératiniques, en particulier les cils et/ou les sourcils et de préférence les sourcils, étant ensuite, dans une étape finale et de façon optionnelle, rincées.

Selon un mode particulier et préféré, la composition A colorante est appliquée avant la composition B révélatrice.

Selon un premier mode, la composition A colorante est appliquée sur des fibres kératiniques qui ne sont pas préalablement traitées par un agent oxydant.

Selon un autre mode, la composition A colorante est appliquée sur des fibres kératiniques préalablement traitées par un agent oxydant, de préférence du peroxyde d'hydrogène. Le temps de pré-traitement à l'agent oxydant ira généralement de 30 secondes à 10 minutes, de préférence de 30 secondes à 5 minutes. Et selon un mode particulier, une étape de rinçage intermédiaire et éventuellement séchage est réalisée entre l'étape de pré-traitement à l'agent oxydant et l'étape de traitement avec la composition colorante.

### Kit ou dispositif

L'invention porte également sur un kit ou dispositif de coloration ou maquillage des fibres kératiniques, en particulier les cils et/ou les sourcils et de préférence les sourcils, à plusieurs compartiments séparés et comprenant :
- dans un compartiment, la composition A colorante telle que définie dans l'invention;
- dans un autre compartiment séparé la composition B révélatrice telle que définie
   dans l'invention, et caractérisée en ce qu'elle ne comprend pas de sel de fer
   et/ou d'aluminium, en particulier le gluconate de fer, et
- dans un autre compartiment séparé, le gluconate de fer destiné à être mélangé extemporanément à la composition B révélatrice avant application sur les fibres kératiniques,
- de façon optionnelle, dans un autre compartiment séparé une composition C comprenant un agent oxydant pour un pré-traitement des fibres kératiniques avant traitement par les compositions A et B,
- et avantageusement une notice d'utilisation.

Selon un mode alternatif, le kit ou dispositif selon l'invention comprend :
- dans un compartiment, la composition A colorante telle que définie dans l'invention;
- dans un autre compartiment séparé la composition B révélatrice telle que définie dans l'invention et caractérisée en ce qu'elle comprend le gluconate de fer,
- de façon optionnelle, dans un autre compartiment séparé une composition C comprenant un agent oxydant pour un pré-traitement des fibres kératiniques avant traitement par les compositions A et B,
- et avantageusement une notice d'utilisation.

Selon un mode particulier et préféré, le kit ou dispositif selon l'invention comprend :
- la composition A colorante comprenant
   i. un colorant naturel choisi dans le groupe constitué par un extrait de campêche, un extrait de châtaignier, un extrait de Sorgho, un extrait de pin, un extrait de thé, un extrait de noix de Galle, un extrait de cacao, un extrait de Brasiletto, et leurs mélanges ;
   ii. un decyl glucoside comme tensioactif non ionique ;
   iii. un sodium metabisulfite comme agent antioxydant ;
   iv. un polymère acrylique comme gélifiant,
   v. un ajusteur de pH et
   vi. optionnellement des pigments noirs et/ou colorants directs cationiques;
- la composition B révélatrice comprenant
   vii. un polymère acrylique comme gélifiant,
   viii. un ajusteur de pH, et
   ix. optionnellement un polymère filmogène,
- une composition séparée comprenant le gluconate de fer comme sel de fer, et
- optionnellement une composition séparée comprenant le peroxyde d'hydrogène comme agent oxydant optionnel.

Les compositions A et B sont telles que décrites précédemment.

Selon un mode particulier, les compositions A et B sont sous forme de gel aqueux, la composition A comprenant une phase aqueuse gélifiée par un polymère carboxyvinylique et la composition B comprenant une phase aqueuse gélifiée par un polymère associatif, en particulier un polymère anionique associatif.

Les compositions du kit sont conditionnées dans des compartiments distincts, accompagnés, éventuellement, de moyens d'application appropriés, identiques ou différents, tels que les pinceaux, les brosses ou les éponges.

Selon un mode particulier et préféré, le kit comprend un pinceau ou une brosse pour application des compositions de l'invention.

La présente invention va désormais être illustrée par les exemples non limitatifs suivants. Sauf indication contraire, les % sont exprimés en % en poids par rapport au poids total de ladite composition.

### EXEMPLES

### Exemple 1 : Formulations pour coloration des sourcils sous forme de gels

### Première composition comprenant l'extrait de campêche

| | |
|---|---|
| Extrait de bois de campêche* | 5% |
| Glycerol | 5% |
| Dimethyl isosorbide (DMI) | 1% |
| Carbomer (Carbopol Ultrez 30) | 1,2% |
| Alkyl glucoside | 4% |
| Soude (solution à 10%) | 7% |
| Alcool benzylique | 0,8% |
| Métabisulfite de sodium | 0,05% |
| Conservateurs | qs |
| Eau déminéralisée | qsp 100% |
| *Logwood extract de la société SCRD | |

La composition A colorante est préparée selon le mode opératoire suivant :
- le diméthyl isosorbide, le glycérol, le métabisulfite de sodium et les conservateurs sont mélangés à de l'eau sous agitation ;
- le carbomer (gélifiant) est ensuite ajouté à la phase précédente et mélangé jusqu'à dissolution complète ;
- on ajoute ensuite la solution de soude à 10% ; la formule s'épaissit immédiatement ;
- on ajoute alors l'extrait de bois de campêche et on mélange jusqu'à dissolution complète ;
- on ajoute enfin le tensioactif alkyl glucoside et de l'eau et on homogénéise le gel ainsi obtenu ;
- on vérifie que le pH se situe entre 7 et 7,5.

### Composition B révélatrice comprenant le gluconate de fer

| | |
|---|---|
| Polymère épaississant polyacrylate | |
| (Polyacrylate Crosspolymer-6, SEPIMAX ZEN^{®} de SEPPIC) | 0,5% |
| Carbonate de sodium | 0,5% |
| Alcool benzylique | 0,8% |
| Conservateurs | qs |
| Eau déminéralisée | qsp 100% |
| | |
| Gluconate de fer | 3,6% |

La composition B révélatrice est préparée selon le mode opératoire suivant :
- les ingrédients, à l'exception du gélifiant et du gluconate de fer sont mélangés sous agitation jusqu'à parfaire dissolution ;
- on ajoute ensuite le gélifiant jusqu'à parfaite dissolution et formation du gel ;
- le gluconate de fer est ajouté extemporanément à la composition B, avant application du produit sur les sourcils

L'application sur les sourcils est réalisée selon le mode opératoire suivant :
- on applique la composition A colorante sur les sourcils à l'aide d'un pinceau ou d'une brosse, avec un temps de pose de 4 à 6 minutes ;
- on applique ensuite la composition B révélatrice sur les sourcils à l'aide d'un pinceau ou d'une brosse, avec un temps de pose de 2 minutes ;
- on rince ensuite les sourcils.

On obtient une couleur brune sur les sourcils avec une bonne tenue dans le temps.

### Exemple 2 : Sélection d'agents booster de pénétration, seuls ou en combinaison, sur la tenue de la couleur sur le sourcil

La Demanderesse a montré que l'utilisation d'un ou plusieurs agent(s) booster de pénétration permettait d'améliorer le résultat couleur sur les sourcils et sa tenue dans le temps. Elle a testé différents agents parmi lesquels les phospholipides, les céramides, les saponines, les acides gras et sélectionné parmi les agents les plus performants notamment le dimethyl isosorbide (Arlasolve DMI de Univar) et le PEG-7 Glyceryl Cocoate (Cetiol^{®} HE de BASF).

Les résultats présentés ci-dessous illustrent l'effet respectivement de phospholipides et du diméthyl isosorbide.

### 2.1 Effet du dimethyl isosorbide comparativement à des phospholipides

Différentes compositions sont testées sur un panel de 10 personnes.
Composition A1 : extrait de campêche + DMI 2%
Composition A2 : extrait de campêche + colorants cationiques (faible quantité) sans DMI
Composition A3 : extrait de campêche + lécithine (phospholipide) 1%
Composition B : comprenant le gluconate de fer

Les compositions A1 à A3 sont préparées selon l'exemple 1 et la composition B correspond à l'exemple décrit à l'exemple 1.

Les compositions A1 à A4 sont appliquées 4 minutes.

La composition B est appliquée 2 minutes.

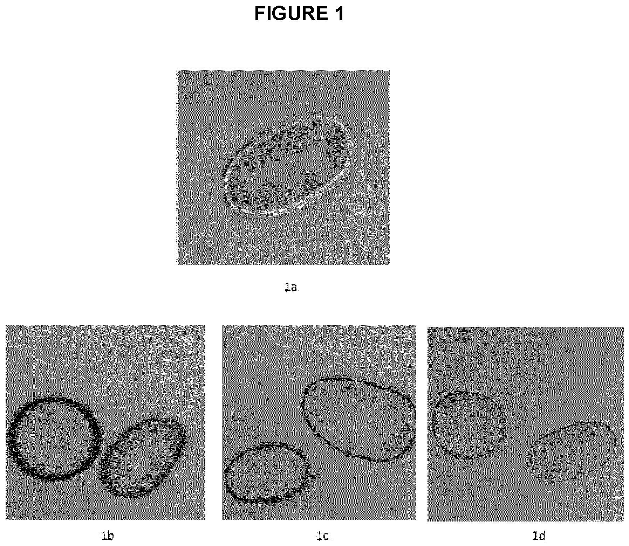

On évalue l'effet de la présence ou non de l'agent booster de pénétration de la couleur dans la composition A colorante en réalisant des observations au microscope sur des coupes de fibres kératiniques traitées ou non traitées par lesdites compositions (figure 1). Une fibre non traitée est caractérisée par un halo blanc (pourtour blanc) alors qu'une fibre traitée (colorée) est caractérisée par un halo noir. Plus ce halo est épais, plus la diffusion de la couleur dans la fibre kératinique est profonde.

[Fig. 1] Les résultats sont présentés à la Figure 1 : la figure 1a correspond à la condition non traitée ; on observe un halo coloré plus épais sur la figure 1b (traité avec une composition A1 comprenant DMI), comparativement aux traitements avec des colorants cationiques sans DMI (figure 1c) ou des phospholipides (figure 1d).

Ces résultats montrent que le dimethyl isosorbide favorise et/ou augmente la pénétration du colorant naturel (campêche) dans les fibres kératiniques de façon améliorée comparativement aux phospholipides.

### 2.2 Effet du dimethyl isosorbide (DMI)

Des compositions similaires à celles décrites à l'exemple 1 sont reproduites ; on utilise deux colorants naturels en une teneur totale de 5%, à savoir un extrait de campêche (Logwood extract de SCRD) et un extrait de châtaignier (par exemple, celui de la société Couleurs de plantes) dans une proportion 60/40 (60% d'extrait de campêche et 40% d'extrait de châtaignier).

Dans la composition comparative (sans DMI), le DMI est remplacé par de l'eau.

On évalue à T0, T3, T7, T9 et T21 (exprimés en jours) les deux compositions selon les paramètres de couleur L (intensité) et delta E1 (tenue de la couleur dans le temps) à l'aide et de l'appareil de capture photo : Visia et du logiciel d'analyse d'image: Visilog.

On obtient des valeurs pour les paramètres colorimétriques :
« L* » qui correspond à la luminosité de couleur du sourcil
« a* » (composante Vert / Rouge) et « b* » (composante Bleu / Jaune) qui correspondent à la couleur du sourcil

On mesure respectivement :
- la différence de perception de couleur par rapport au temps « 0 » (= temps avant application) : Delta E0 (ΔE0) = √((L0-Li)² + (a0-ai)² + (b0-bi)²) et
- la différence de perception de couleur par rapport au temps « 1 » (= temps immédiat après application) : Delta E1 (ΔE1) = √((L1-Li)² + (a1-ai)² + (b1-bi)²)

On procède ensuite à l'analyse statistique de ces paramètres (L*, Delta E0 et DeltaE1).

La tenue du produit est validée lorsqu'il y a une différence entre le temps avant application et les autres temps de mesures pour les paramètres L* et DeltaE1.

On fait ensuite le calcul du pourcentage de perte de la luminosité L* par rapport au temps immédiat après application : % = (Après-Avant) / Avant * 100

On obtient les résultats suivants :
- Evaluation de l'intensité de la couleur (paramètre L) : Après application, il n'y a pas de différence en termes d'intensité entre les deux compositions. A T3, T7, T9 jours, la composition selon l'invention avec DMI est plus intense comparativement à la composition sans DMI. A T21, cette différence est encore mesurable.
- Evaluation de la tenue de la couleur dans le temps (paramètre deltaE1) : Après application, il n'y a pas de différence en terme de couleur entre les deux compositions. A T3, T7, T9, T21 jours, la composition comparative sans DMI présente une variation de couleur (delta E1) plus importante que la composition avec DMI, qui permet donc une meilleure tenue de la couleur dans le temps.

### 2.3 Effet du PEG-7 Glyceryl Cocoate

On reproduit l'exemple 2.1 en remplaçant le DMI par 1% de PEG-7 Glyceryl Cocoate (Cetiol HE).

La composition A colorante est appliquée avec un temps de pose de 6 minutes. Selon une alternative, elle est appliquée avec un temps de pose de 3 minutes.

On observe des variations similaires (paramètres L et delta E1) à celles observées avec le DMI, avec des résultats améliorés avec le temps de pose de 6 minutes : le résultat intensité de la couleur et tenue dans le temps tient 21 jours avec le temps de pose de 6 minutes, contre 9 jours avec le temps de pose de 3 minutes.

### Exemple 3 : Effet d'un pré-traitement oxydant sur le résultat coloration ou maquillage

Différentes compositions sont testées, selon le modèle décrit à l'exemple 1 :
Composition A1 : extrait de campêche + DMI 1%
Composition A2 : extrait de campêche + Cetiol HE 1%
Composition A3 : extrait de campêche seul
Composition A4 : extrait de campêche + DMI 1% + Cetiol HE 1%
Composition B : comprenant le gluconate de fer

Les compositions A1 à A4 sont préparées selon l'exemple 1 et la composition B correspond à l'exemple décrit à l'exemple 1.

Les compositions A1 à A4 sont appliquées 4 ou 6 minutes.

La composition B est appliquée 2 minutes.

On teste ces différentes compositions avec ou sans pré-traitement des fibres kératiniques (cheveux et sourcils) avec un agent oxydant, en particulier le peroxyde d'hydrogène à 12%. Le pré-traitement à l'agent oxydant est réalisé pendant 2 minutes ou pendant 10 minutes. Ce pré-traitement oxydant va favoriser l'ouverture des écailles des fibres kératiniques et permettre ainsi une meilleure pénétration des agents colorants.

On évalue l'effet du pré-traitement oxydant et la présence ou non de l'agent booster de pénétration de la couleur dans la composition A colorante en réalisant des observations au microscope sur des coupes de fibres kératiniques traitées/ non traitées. Une fibre non traitée par l'agent oxydant est caractérisée par un halo blanc (pourtour blanc) alors qu'une fibre traitée (colorée) est caractérisée par un halo noir. Plus ce halo est épais, plus la diffusion de la couleur dans la fibre kératinique est profonde. On mesure ainsi le pourcentage de sourcils colorés (par comptage des sourcils colorés) pour chaque condition testée et la diffusion de la couleur (épaisseur du halo) en utilisant un logiciel MatLab, pour les sourcils pré-traités à l'agent oxydant (les sourcils non pré-traités à l'agent oxydant ne permettent pas une telle évaluation, leur couleur naturelle foncée- du fait de la présence de mélanine- ne permettant pas d'objectiver une différence de couleur).

Les résultats (% de sourcils colorés) sont présentés dans le tableau 1 suivant :

**Tableau 1**

| | Sans pré-traitement oxydant | | Avec pré-traitement oxydant | |
|---|---|---|---|---|
| Temps de pose des compositions A et B | 4 min A puis 2 min B | 6 min A puis 2 min B | 4 min A puis 2 min B | 6 min A puis 2 min B |
| Composition A3 (sans booster de pénétration) | 27 % | 58 % | 100 % | 80 % |
| Composition A1 (DMI 1%) | 50 % | 53 % | 100 % | 86 % |
| Composition A2 ( CETIOL HE 1%) | 50 % | nd | 100 % | 100 % |
| Composition A4 (DMI 1% + CETIOL HE 1%) | 40 % | 74 % | 100 % | 100 % |

| | | | | |
|---|---|---|---|---|
| nd= non détermine (non mesuré) | | | | |

Ces résultats montrent que le pré-traitement des sourcils avec un agent oxydant, avant application des compositions A et B, permet d'augmenter l'efficacité de coloration : le nombre de sourcils colorés après ce pré-traitement à l'agent oxydant est de 100% pour quasiment toutes les conditions, alors que sans pré-traitement oxydant, une proportion des sourcils ne sont pas colorés (auréole noire non détectable au microscope).

L'utilisation de la combinaison de DMI et Cetiol HE avec un temps de pause de 6 minutes pour la composition A et 2 minutes pour la composition B donne les meilleurs résultats, que la fibre kératinique soit ou non traitée en amont par un agent oxydant.

Une évaluation au microscope de l'épaisseur des halo colorés (en µm), représentative de la diffusion de la couleur dans la fibre est réalisée sur les sourcils pré-traités avec l'agent oxydant. Comme indiqué ci-dessus, les sourcils non pré-traités à l'agent oxydant ne permettent pas une telle évaluation, leur couleur naturelle foncée- du fait de la présence de mélanine- ne permettant pas d'objectiver une différence de couleur. On obtient les résultats suivants présentés dans le tableau 2 :

**Tableau 2**

| | Pré-traitement oxydant | |
|---|---|---|
| Temps de pose des compositions A et B | 4 min A puis 2 min B | 6 min A puis 2 min B |
| Composition A3 (sans booster de pénétration) | 4.77 µm +/- 1.86 | 2.74 µm +/- 0.75 |
| Composition A1 (DMI 1%) | 3.68 µm +/- 0.72 | 3.66 µm +/- 0.77 |
| Composition A2 ( CETIOL HE 1%) | 3.99 µm +/- 1.31 | 5.52 µm +/- 1.16 |
| Composition A4 (DMI 1% + CETIOL HE 1%) | 3.76 µm +/- 0.30 | 6.2 µm +/-1.87 |

Ces résultats confirment que la meilleure condition pour une coloration optimale des sourcils est la condition DMI 1% + CETIOL HE 1% avec un temps de pose de la composition A colorante de 6 minutes et un temps de pose de la composition B révélatrice de 2 minutes.

### Exemple 4 : Sélection d'un gélifiant anionique associatif pour améliorer la stabilité de la composition B révélatrice sous forme de gel

La Demanderesse a testé différents gélifiants polymériques hydrophiles et mis en évidence qu'un polymère anionique associatif permettait d'améliorer la stabilité de la composition B révélatrice au contact du gluconate de fer. L'objectif de cette étude était de sélectionner des gélifiants permettant d'avoir une viscosité acceptable, et qui ne se « casse » pas lors de l'ajout du gluconate de fer. Des compositions B (révélatrices) telles que décrites à l'exemple 1 ont été préparées, respectivement avec les gélifiants suivants :

| | |
|---|---|
| Gélifiant 1 : | Polyacrylamide & C13-14 Isoparaffin & Laureth-7 (Sepigel 305^{™}) testé à 3 et 5% |
| Gélifiant 2 : | Hydroxyethyl Acrylate/Sodium Acryloyldimethyl Taurate Copolymer (Sepinov^{™} EMT10) testé à 4% |
| Gélifiant 3 : | Hydroxyethyl cellulose (Natrosol 250 HR) testé de 0.5 à 4% |
| Gélifiant 4 : | Polyacrylate Crosspolymer-6 (SEPIMAX ZEN^{®}) testé de 0.5 à 3%. |

Les ingrédients de chaque composition B, à l'exception du gélifiant et du gluconate de fer sont mélangés sous agitation jusqu'à parfaire dissolution ; on ajoute ensuite le gélifiant jusqu'à parfaite dissolution et formation du gel ; le gluconate de fer est ensuite ajouté extemporanément à la composition B, avant application du produit sur les sourcils.

Les gélifiants 1, 2 et 3 ne permettent pas au gel de résister lors de l'ajout du gluconate de fer : le gel devient liquide et n'est donc plus applicable. En revanche, le gélifiant 4 de type Polyacrylate Crosspolymer-6 permet lui de maintenir une composition B stable lors de l'ajout du gluconate de fer.

### Exemple 5 : Formulations et mise en oeuvre du procédé

### 5.1 Composition A (colorante) avec un extrait de campêche

| | |
|---|---|
| Extrait de bois de campêche* | 5% |
| Glycerol | 5% |
| Dimethyl isosorbide (DMI) | 1% |
| Carbomer (Carbopol Ultrez 30) | 1,2% |
| Alkyl glucoside | 4% |
| Soude (solution à 10%) | 7% |
| Alcool benzylique | 0,8% |
| Métabisulfite de sodium | 0,05% |
| Conservateurs | qs |
| Eau déminéralisée | qsp 100% |
| *Logwood extract de la société SCRD | |

La composition A colorante est préparée selon le mode opératoire suivant :
- le diméthyl isosorbide, le glycérol, le métabisulfite de sodium et les conservateurs sont mélangés à de l'eau sous agitation ;
- le carbomer (gélifiant) est ensuite ajouté à la phase précédente et mélangé jusqu'à dissolution complète ;
- on ajoute ensuite la solution de soude à 10% ; la formule s'épaissit immédiatement ;
- on ajoute alors l'extrait de bois de campêche et on mélange jusqu'à dissolution complète ;
- on ajoute enfin le tensioactif alkyl glucoside et de l'eau et on homogénéise le gel ainsi obtenu ;
- on vérifie que le pH se situe entre 7 et 7,5.

### 5.2 Composition A (colorante) avec un extrait de châtaignier et un extrait de campêche

| | |
|---|---|
| Extrait de châtaignier | 2,5% |
| Extrait de bois de campêche* | 2,5% |
| Glycerol | 5% |
| Dimethyl isosorbide (DMI) | 1% |
| Carbomer (Carbopol Ultrez 30) | 1,2% |
| Alkyl glucoside | 4% |
| Soude (solution à 10%) | 7% |
| Alcool benzylique | 0,8% |
| Métabisulfite de sodium | 0,05% |
| Conservateurs | qs |
| Eau déminéralisée | qsp 100% |
| *Logwood extract de la société SCRD | |

On prépare la composition A selon le protocole décrit ci-dessus.

### 5.3 Composition A (colorante) avec un extrait de bois rouge, un extrait de châtaignier et un extrait de campêche

| | |
|---|---|
| Extrait de Brasiletto (société SCRD) | 1,66% |
| Extrait de châtaignier | 1,66% |
| Extrait de bois de campêche* | 1,66% |
| Glycerol | 5% |
| Dimethyl isosorbide (DMI) | 1% |
| Carbomer (Carbopol Ultrez 30) | 1,2% |
| Alkyl glucoside | 4% |
| Soude (solution à 10%) | 7% |
| Alcool benzylique | 0,8% |
| Métabisulfite de sodium | 0,05% |
| Conservateurs | qs |
| Eau déminéralisée | qsp 100% |
| *Logwood extract de la société SCRD | |

### 5.4 Composition B révélatrice comprenant le gluconate de fer

Polymère épaississant polyacrylate

| | |
|---|---|
| (Polyacrylate Crosspolymer-6, SEPIMAX ZEN^{®} de SEPPIC) | 0,5% |
| Carbonate de sodium | 0,5% |
| Alcool benzylique | 0,8% |
| Conservateurs | qs |
| Eau déminéralisée | qsp 100% |
| Gluconate de fer | 3,6% |

La composition B révélatrice est préparée selon le mode opératoire suivant :
- les ingrédients, à l'exception du gélifiant et du gluconate de fer sont mélangés sous agitation jusqu'à parfaire dissolution ;
- on ajoute ensuite le gélifiant jusqu'à parfaite dissolution et formation du gel ;
- le gluconate de fer est ajouté extemporanément à la composition B, avant application du produit sur les sourcils

### 5.5 Procédé de coloration ou maquillage des sourcils

Le protocole de coloration ou maquillage des sourcils mettant en oeuvre successivement la composition A et la composition B sus-décrites est le suivant :
1/ Nettoyer les sourcils à l'eau micellaire
2/ Essuyer avec un coton imbibé d'eau
3/ Brosser les sourcils avec un goupillon
4/ Essuyer le sourcil pour enlever toute trace d'humidité à l'aide d'un mouchoir en papier
5/Appliquer de la vaseline en couche très épaisse autour du sourcil pour ne pas teinter la peau
6/ Appliquer la composition A en couche épaisse en suivant bien la ligne du sourcil de façon à bien recouvrir tous les poils (Temps de pose : 4 min. Ne pas essuyer le produit) 7/ Mélange de la composition B : ajouter le Gluconate de fer dans le pot blanc contenant la composition B et mélanger jusqu'à ce qu'il n'y ait plus de grain 8/ Appliquer la composition B ainsi préparée extemporanément en couche épaisse pardessus la composition A, en suivant bien la ligne du sourcil de façon à bien recouvrir tous les poils (Temps de pose : 2 min. Pendant ce temps, rectifier si besoin le contour du sourcil à l'aide d'un coton tige).
9/ A la fin des 2 min de pose, essuyer l'excès de produit à l'aide d'un coton sec suivit d'un coton humidifié avec de l'eau (brume) Retouches possibles.

On obtient une couleur brune sur les sourcils avec une bonne tenue dans le temps.

## Revendications

1. Procédé cosmétique de coloration ou maquillage des fibres kératiniques, en particulier les cils et/ou les sourcils et de préférence les sourcils, comprenant l'application sur lesdites fibres kératiniques, d'au moins :
a) Une composition cosmétique A colorante sous forme de gel aqueux comprenant, dans un milieu physiologiquement acceptable, un gélifiant polymérique acrylique, un ou plusieurs colorant(s) naturel(s) choisi(s) dans le groupe constitué par les néoflavonoïdes, les tanins galliques et tanins catéchiques, les proanthocyanidines, et leurs mélanges, ou de préférence un ou plusieurs extraits végétaux en contenant, et avantageusement un agent antioxydant,
b) Une composition cosmétique B révélatrice sous forme de gel aqueux comprenant, dans un milieu physiologiquement acceptable, un gélifiant polymérique choisi parmi les polymères anioniques associatifs, et du gluconate de fer,
la composition A étant appliquée avant ou après la composition B

2. Procédé cosmétique selon la revendication 1, dans lequel la composition A est appliquée sur fibres kératiniques, en particulier les cils et/ou les sourcils et de préférence les sourcils, avant la composition B.

3. Procédé cosmétique selon la revendication 1 ou la revendication 2, dans lequel le gluconate de fer est ajouté extemporanément à la composition B révélatrice, avant application de celle-ci sur lesdites fibres kératiniques, en particulier les cils et/ou les sourcils et de préférence les sourcils.

4. Procédé cosmétique selon l'une quelconque des revendications précédentes, dans lequel la composition A colorante comprend au moins un colorant choisi parmi l'hématoxyline, l'hématéine, la braziléine, la braziline, l'acide gallique, la catéchine, la castalagine, la vescalagine, et/ou les procyanidines et leurs mélanges, ou un extrait végétal en contenant, en particulier un extrait végétal choisi dans le groupe constitué par un extrait de campêche, un extrait de bois rouge, un extrait de châtaignier, un extrait de noix de Galle, un extrait d'écorce d'Anogeissus un extrait de pin, un extrait de thé, un extrait de vigne, un extrait de Sorgho, un extrait de cacao, un extrait de myrobalan, un extrait d'hibiscus, un extrait de fraisier et leurs mélanges, de préférence un extrait de campêche, un extrait de bois rouge, un extrait de châtaignier, et leurs mélanges.

5. Procédé cosmétique selon l'une quelconque des revendications précédentes, dans lequel le ou les colorants naturels ou le ou les extraits végétaux en contenant sont présents en une teneur totale allant de 0,1 à 10%, de préférence de 2 à 5% en poids par rapport au poids total de la composition.

6. Procédé cosmétique selon l'une quelconque des revendications précédentes, dans lequel la composition A colorante comprend en outre un tensioactif non ionique, en particulier un alkyl glucoside, de préférence le decyl glucoside.

7. Procédé cosmétique selon l'une quelconque des revendications précédentes, dans lequel le gélifiant polymérique acrylique dans la composition A colorante est choisi parmi les polymères ou copolymères d'acides acryliques et méthacryliques comme les copolymères acide acrylique/acrylate d'éthyle et les polymères carboxyvinyliques.

8. Procédé cosmétique selon l'une quelconque des revendications précédentes, dans lequel la composition A colorante comprend en outre au moins une matière colorante additionnelle choisie parmi les pigments, les colorants et leurs mélanges, en particulier un colorant direct, de préférence un colorant cationique direct.

9. Procédé cosmétique selon l'une quelconque des revendications précédentes, dans lequel la composition B révélatrice comprend au moins un polymère filmogène.

10. Procédé cosmétique selon l'une quelconque des revendications précédentes, dans lequel le polymère anionique associatif dans la composition B révélatrice est choisi parmi les terpolymères anioniques, de préférence un terpolymère anionique d'acide 2-méthyl-2-[(1-oxo-2-propènyl]amino] 1-propanesulfonique partiellement ou totalement salifié sous forme de sel d'ammonium, de N,N-diméthylacrylamide et d'acrylate de lauryle tétraéthoxylé et réticulé au triméthylol propanetriacrylate, de nom INCI Polyacrylate Crosspolymer-6.

11. Procédé cosmétique selon la revendication précédente, dans lequel le polymère anionique associatif dans la composition B révélatrice est présent en une teneur allant de 0,1 à 5%, de préférence de 0,5% à 3%, en poids par rapport au poids total de ladite composition.

12. Procédé cosmétique selon l'une quelconque des revendications précédentes, dans lequel :
- la composition A colorante est appliquée sur les fibres kératiniques, en particulier les cils et/ou les sourcils et de préférence les sourcils, avec un temps de pose allant de 30 secondes à 30 minutes, préférentiellement de 45 secondes à 10 minutes, préférentiellement de 1 minutes à 6 minutes,
- la composition B révélatrice est appliquée les fibres kératiniques, en particulier les cils et/ou les sourcils et de préférence les sourcils, avec un temps de pose allant de 30 secondes à 10 minutes, préférentiellement de 45 secondes à 5 minutes,
la composition A étant appliquée avant ou après la composition B, de préférence avant la composition B,
- lesdites fibres kératiniques, en particulier les cils et/ou les sourcils et de préférence les sourcils, étant ensuite, dans une étape finale et de façon optionnelle, rincées.

13. Kit ou dispositif de coloration ou maquillage des fibres kératiniques, en particulier les cils et/ou les sourcils et de préférence les sourcils, à plusieurs compartiments séparés et comprenant :
- dans un compartiment, la composition A colorante telle que définie dans l'une quelconque des revendications 1 et 4 à 8 ;
- dans un autre compartiment séparé la composition B révélatrice telle que définie dans l'une quelconque des revendications 1 et 9 à 11 et **caractérisée en ce qu'**elle ne comprend pas de gluconate de fer, et
- dans un autre compartiment séparé, du gluconate de fer destiné à être mélangé extemporanément à la composition B révélatrice avant application sur les fibres kératiniques,
- et avantageusement une notice d'utilisation.

14. Kit ou dispositif de coloration ou maquillage des fibres kératiniques, en particulier les cils et/ou les sourcils et de préférence les sourcils, à plusieurs compartiments séparés et comprenant :
- dans un compartiment, la composition A colorante telle que définie dans l'une quelconque des revendications 1 et 4 à 8 ;
- dans un autre compartiment séparé la composition B révélatrice telle que définie dans l'une quelconque des revendications 1 et 9 à 11 et **caractérisée en ce qu'**elle comprend du gluconate de fer,
- et avantageusement une notice d'utilisation.

15. Kit ou dispositif selon la revendication 13 ou la revendication 14 dans lequel :
- la composition A colorante comprend
i. un colorant naturel choisi dans le groupe constitué par un extrait de campêche, un extrait de châtaignier, un extrait de Sorgho, un extrait de pin, un extrait de thé, un extrait de noix de Galle, un extrait de cacao, un extrait de Brasiletto, et leurs mélanges ;
ii. un decyl glucoside comme tensioactif non ionique ;
iii. un sodium metabisulfite comme agent antioxydant ;
iv. un polymère acrylique comme gélifiant,
v. un ajusteur de pH et
vi. optionnellement des pigments noirs et/ou colorants directs cationiques;
- la composition B révélatrice comprend
vii. un polymère anionique associatif choisi parmi les terpolymères anioniques, de préférence un terpolymère anionique d'acide 2-méthyl-2-[(1-oxo-2-propènyl]amino] 1-propanesulfonique partiellement ou totalement salifié sous forme de sel d'ammonium, de N,N-diméthylacrylamide et d'acrylate de lauryle tétraéthoxylé et réticulé au triméthylol propanetriacrylate, de nom INCI Polyacrylate Crosspolymer-6 comme gélifiant,
viii. un ajusteur de pH, et
ix. optionnellement un polymère filmogène.

## Patentansprüche

1. Kosmetisches Verfahren zum Färben oder Schminken der Keratinfasern, vor allem der Wimpern und/oder der Augenbrauen und vorzugsweise der Augenbrauen, welches das Auftragen auf die Keratinfasern umfasst von mindestens:
a) einer färbenden kosmetischen Zusammensetzung A in Form eines wässrigen Gels, das in einem physiologisch akzeptablen Medium einen Acrylpolymergelbildner, einen oder mehrere natürliche Farbstoffe, die aus der Gruppe ausgewählt sind, die von den Neoflavonoiden, den Gallotanninen und kondensierten Tanninen, den Proanthocyanidinen und deren Gemischen oder vorzugsweise einem oder mehreren Pflanzenextrakten, die davon enthalten, gebildet ist und in vorteilhafter Weise ein Antioxidationsmittel umfasst,
b) eine entwickelnde kosmetischen Zusammensetzung B in Form eines wässrigen Gels, das in einem physiologisch akzeptablen Medium einen Polymergelbildner, der aus den assoziativen anionischen Polymeren ausgewählt ist, und Eisengluconat umfasst,
wobei die Zusammensetzung A vor oder nach der Zusammensetzung B aufgetragen wird.

2. Kosmetisches Verfahren nach Anspruch 1, wobei die Zusammensetzung A auf Keratinfasern, vor allem die Wimpern und/oder die Augenbrauen und vorzugsweise die Augenbrauen, vor der Zusammensetzung B aufgetragen wird.

3. Kosmetisches Verfahren nach Anspruch 1 oder Anspruch 2, wobei das Eisengluconat der entwickelnden Zusammensetzung unmittelbar vor dem Auftragen derselben auf die Keratinfasern, vor allem die Wimpern und/oder die Augenbrauen und vorzugsweise die Augenbrauen, hinzugefügt wird.

4. Kosmetisches Verfahren nach einem der vorangehenden Ansprüche, wobei die färbende Zusammensetzung A mindestens einen Farbstoff umfasst, der aus Hämatoxylin, Hämatein, Brazilein, Brazilin, Gallussäure, Catechin, Castalagin, Vescalagin und/oder den Procyanidinen und ihren Gemischen ausgewählt ist oder einen Pflanzenextrakt, der davon enthält, vor allem einen Pflanzenextrakt, der aus der Gruppe ausgewählt ist, die von einem Blutholzbaumextrakt, einem Rotholzbaumextrakt, einem Kastanienbaumextrakt, einem Gallapfelextrakt, einem Anogeissusrindenextrakt, einem Kiefernextrakt, einem Teeextrakt, einem Weinstockextrakt, einem Sorghumextrakt, einem Kakaoextrakt, einem Myrobalanenextrakt, einem Hibiskusextrakt, einem Erdbeerpflanzenextrakt und ihren Gemischen gebildet ist, vorzugsweise einen Blutholzbaumextrakt, einen Rotholzbaumextrakt, einen Kastanienbaumextrakt und ihre Gemische.

5. Kosmetisches Verfahren nach einem der vorangehenden Ansprüche, wobei der oder die natürlichen Farbstoffe oder der oder die Pflanzenextrakte, die davon enthalten, in einem Gesamtgehalt von 0,1 bis 10, vorzugsweise von 2 bis 5 Gew.-% im Verhältnis zum Gesamtgewicht der Zusammensetzung vorhanden sind.

6. Kosmetisches Verfahren nach einem der vorangehenden Ansprüche, wobei die färbende Zusammensetzung A ferner ein nichtionisches Tensid, vor allem ein Alkylglucosid, vorzugsweise Decylglucosid, umfasst.

7. Kosmetisches Verfahren nach einem der vorangehenden Ansprüche, wobei der Acrylpolymergelbildner in der färbenden Zusammensetzung A aus den Acryl- und Methacrylsäurepolymeren oder -copolymeren wie die Acrylsäure-/Ethylacrylatcopolymere und die Carboxyvinylpolymere ausgewählt ist.

8. Kosmetisches Verfahren nach einem der vorangehenden Ansprüche, wobei die färbende Zusammensetzung A ferner mindestens ein zusätzliches Färbemittel umfasst, das aus den Pigmenten, den Farbstoffen und ihren Gemischen ausgewählt ist, vor allem einen direkten Farbstoff, vorzugsweise einen direkten kationischen Farbstoff.

9. Kosmetisches Verfahren nach einem der vorangehenden Ansprüche, wobei die entwickelnde Zusammensetzung B mindestens ein filmbildendes Polymer umfasst.

10. Kosmetisches Verfahren nach einem der vorangehenden Ansprüche, wobei das assoziative anionische Polymer in der entwickelnden Zusammensetzung B aus den anionischen Terpolymeren ausgewählt ist, vorzugsweise ein anionisches 2-Methyl-2-[(1-oxo-2-propenyl]amino] 1-propansulfonsäure-Terpolymer, teilweise oder vollständig versalzt in Form eines Ammoniumsalzes, von N,N-Dimethylacrylamid und tetraethoxyliertem Laurylacrylat und vernetzt mit Trimethylolpropantriacrylat mit der INCI-Bezeichnung Polyacrylate Crosspolymer-6.

11. Kosmetisches Verfahren nach vorangehendem Anspruch, wobei das assoziative anionische Polymer in der entwickelnden Zusammensetzung B in einem Gehalt von 0,1 bis 5, vorzugsweise von 0,5 bis 3 Gew.-% im Verhältnis zum Gesamtgewicht der Zusammensetzung vorhanden ist.

12. Kosmetisches Verfahren nach einem der vorangehenden Ansprüche, wobei:
- die färbende Zusammensetzung A auf die Keratinfasern, vor allem die Wimpern und/oder die Augenbrauen und vorzugsweise die Augenbrauen mit einer Wirkzeit von 30 Sekunden bis 30 Minuten, bevorzugt von 45 Sekunden bis 10 Minuten, bevorzugt von 1 Minute bis 6 Minuten aufgetragen wird, wobei die entwickelnde Zusammensetzung B auf die Keratinfasern, vor allem die Wimpern und/oder die Augenbrauen und vorzugsweise die Augenbrauen mit einer Wirkzeit von 30 Sekunden bis 10 Minuten, bevorzugt von 45 Sekunden bis 5 Minuten aufgetragen wird,
wobei die Zusammensetzung A vor oder nach der Zusammensetzung B, vorzugsweise vor der Zusammensetzung B aufgetragen wird
- wobei die Keratinfasern, vor allem die Wimpern und/oder die Augenbrauen und vorzugsweise die Augenbrauen danach in einem abschließenden Schritt und optional abgewaschen werden.

13. Kit oder Vorrichtung zum Färben oder Schminken der Keratinfasern, vor allem der Wimpern und/oder der Augenbrauen und vorzugsweise der Augenbrauen, mit mehreren getrennten Fächern und umfassend:
- in einem Fach die färbende Zusammensetzung A nach einem der Ansprüche 1 und 4 bis 8;
- in einem getrennten anderen Fach die entwickelnde Zusammensetzung B nach einem der Ansprüche 1 und 9 bis 11 und **dadurch gekennzeichnet, dass** sie kein Eisengluconat umfasst, und
- in einem getrennten anderen Fach Eisengluconat, das bestimmt ist, unmittelbar vor dem Auftragen auf die Keratinfasern mit der entwickelnden Zusammensetzung B gemischt zu werden,
- und in vorteilhafter Weise eine Gebrauchsanweisung.

14. Kit oder Vorrichtung zum Färben oder Schminken der Keratinfasern, vor allem der Wimpern und/oder der Augenbrauen und vorzugsweise der Augenbrauen, aus mehreren getrennten Fächern und umfassend:
- in einem Fach die färbende Zusammensetzung A nach einem der Ansprüche 1 und 4 bis 8;
- in einem getrennten anderen Fach die entwickelnde Zusammensetzung B nach einem der Ansprüche 1 und 9 bis 11 und **dadurch gekennzeichnet, dass** sie Eisengluconat umfasst,
- und in vorteilhafter Weise eine Gebrauchsanweisung.

15. Kit oder Vorrichtung nach Anspruch 13 oder Anspruch 14, wobei:
- die färbende Zusammensetzung A umfasst
i. einen natürlichen Farbstoff, der aus der Gruppe ausgewählt ist, die von einem Blutholzbaumextrakt, einem Kastanienbaumextrakt, einem Sorghumextrakt, einem Kiefernextrakt, einem Teeextrakt, einem Gallapfelextrakt, einem Kakaoextrakt, einem Caesalpinienextrakt und ihren Gemischen gebildet ist;
ii. ein Decylglucosid als nichtionisches Tensid;
iii. ein Natriummetabisulfit als Antioxidationsmittel;
iv. ein Acrylpolymer als Gelbildner,
v. einen pH-Regulator und
vi. optional schwarze Pigmente und/oder direkte kationische Farbstoffe;
- die entwickelnde Zusammensetzung B umfasst
vii. ein assoziatives anionisches Polymer, das aus den anionischen Terpolymeren ausgewählt ist, vorzugsweise ein anionisches 2-Methyl-2-[(1-oxo-2-propenyl]amino] 1-propansulfonsäure-Terpolymer, teilweise oder vollständig versalzt in Form von Ammoniumsalz, von N,N-Dimethylacrylamid und tetraethoxyliertem Laurylacrylat und vernetzt mit Trimethylolpropantriacrylat mit der INCI-Bezeichnung Polyacrylate Crosspolymer-6 als Gelbildner,
viii. einen pH-Regulator, und
ix. optional ein filmbildendes Polymer.

## Claims

1. Cosmetic method for dyeing or making up keratin fibres, in particular eyelashes and/or eyebrows and, preferably eyebrows, comprising the application onto said keratin fibres of at least:
a) A cosmetic dyeing composition A in the form of an aqueous gel comprising, in a physiologically-acceptable medium, an acrylic polymeric gelling agent, one or more natural dyes selected from the group consisting of neoflavonoids, gallic tannins and catechetic tannins, proanthocyanidins, and mixtures thereof, or preferably one or more plant extracts containing them, and advantageously an antioxidant agent,
b) A revealing cosmetic composition B in the form of an aqueous gel comprising, in a physiologically-acceptable medium, a polymeric gelling agent chosen from among associative anionic polymers, and iron gluconate,
composition A being applied before or after composition B.

2. Cosmetic method according to claim 1, wherein composition A is applied onto keratin fibres, in particular the eyelashes and/or eyebrows and preferably eyebrows, before composition B.

3. Cosmetic method according to claim 1 or claim 2, wherein iron gluconate is added extemporaneously to revealing composition B before it is applied onto keratin fibres, in particular eyelashes and/or eyebrows and preferably eyebrows.

4. Cosmetic method according to any one of the preceding claims, wherein dyeing composition A comprises at least one dye chosen from among haematoxylin, haematein, brazilein, brazilin, gallic acid, catechin, castalagin, vescalagin, and/or procyanidins and mixtures thereof, or a plant extract containing them, in particular a plant extract chosen from the group consisting of a logwood extract, a Mexican logwood extract, a chestnut extract, a nutgall extract, an *Anogeissus* bark extract, a pine extract, a tea extract, a grapevine extract, a sorghum extract, a cacao extract, a myrobalan extract, a hibiscus extract, a strawberry extract and mixtures thereof, preferably a logwood extract, a Mexican logwood extract, a chestnut extract, and mixtures thereof.

5. Cosmetic method according to any one of the preceding claims, wherein the natural dye(s) or plant extracts containing them are present in a total content ranging from 0.1 to 10%, preferably 2 to 5%, by weight relative to the total weight of the composition.

6. Cosmetic method according to any one of the preceding claims, wherein dye composition A also comprises a nonionic surfactant, in particular an alkyl glucoside, preferably decyl glycoside.

7. Cosmetic method according to any one of the preceding claims, wherein the acrylic polymeric gelling agent in dyeing composition A is chosen from among acrylic acid and methacrylic acid polymers or copolymers such as acrylic acid/ethyl acrylate copolymers and carboxyvinyl polymers.

8. Cosmetic method according to any one of the preceding claims, wherein dyeing composition A also comprises at least one additional coloring material chosen from among pigments, dyes and mixtures thereof, in particular a direct dye, preferably a cationic direct dye.

9. Cosmetic method according to any one of the preceding claims, wherein revealing composition B comprises at least one film-forming polymer.

10. Cosmetic method according to any one of the preceding claims, wherein the associative anionic polymer in revealing composition B is chosen from anionic terpolymers, preferably an anionic terpolymer of 2-methyl-2-[(1-oxo-2-propenyl]amino] 1-propanesulfonic acid partially or totally salified in the form of ammonium salt, N,N-dimethylacrylamide and tetraethoxylated lauryl acrylate crosslinked with trimethylol propanetriacrylate, INCI name Polyacrylate Crosspolymer-6.

11. Cosmetic method according to the preceding claim, wherein the associative anionic polymer in revealing composition B is present in a content ranging from 0.1 to 5%, preferably 0.5% to 3% by weight relative to the total weight of the composition.

12. Cosmetic method according to any one of the preceding claims, wherein:
- dyeing composition A is applied onto keratin fibres, in particular eyelashes and/or eyebrows and preferably eyebrows, with an exposure time ranging from 30 seconds to 30 minutes, preferentially from 45 seconds to 10 minutes, preferentially from 1 minute to 6 minutes,
- revealing composition B is applied onto keratin fibres, in particular eyelashes and/or eyebrows and preferably eyebrows, with an exposure time ranging from 30 seconds to 10 minutes, preferentially from 45 seconds to 5 minutes,
composition A being applied before or after composition B, preferably before composition B,
- said keratin fibres, in particular the eyelashes and/or eyebrows and preferably eyebrows, then being optionally rinsed in a final step.

13. Kit or device for dyeing or making up keratin fibres, in particular eyelashes and/or eyebrows and preferably eyebrows, with several separate compartments and comprising:
- in one compartment, dyeing composition A as defined in any one of claims 1 and 4 to 8;
- in another separate compartment, revealing composition B such as defined in any one of claims 1 and 9 to 11 and **characterized in that** it does not comprise iron gluconate, and
- in another separate compartment, iron gluconate intended to be mixed extemporaneously with revealing composition B before application onto keratin fibres,
- and, advantageously, instructions for use.

14. Kit or device for dyeing or making up keratin fibres, in particular the eyelashes and/or eyebrows and preferably eyebrows, with several separate compartments and comprising
- in one compartment, dyeing composition A such as defined in any one of claims 1 and 4 to 8;
- in another separate compartment revealing composition B as defined in any one of claims 1 and 9 to 11 and **characterized in that** it comprises iron gluconate,
- and, advantageously, instructions for use.

15. Kit or device according to claim 13 or claim 14, wherein:
- dyeing composition A comprises:
i. a natural dye chosen in the group made up of a logwood extract, a chestnut extract, a sorghum extract, a pine extract, a tea extract, a nutgall extract, a cacao extract, a Mexican logwood extract, and mixtures thereof;
ii. a decyl glucoside as a nonionic surfactant;
iii. sodium metabisulfite as an antioxidant;
iv. an acrylic polymer as a gelling agent,
v. a pH adjuster, and
vi. optionally, black pigments and/or cationic direct dyes;
- revealing composition B comprises:
vii. an associative anionic polymer chosen from anionic terpolymers, preferably an anionic terpolymer of 2-methyl-2- [(1-oxo-2-propenyl]amino] 1-propanesulfonic acid partially or totally salified in the form of ammonium salt, N, N-dimethylacrylamide and tetraethoxylated lauryl acrylate crosslinked with trimethylol propanetriacrylate, INCI name Polyacrylate Crosspolymer-6 as gelling agent,
viii. a pH adjuster, and
ix. optionally, a film-forming polymer.
